(19) 

**Europäisches Patentamt**
**European Patent Office**
**Office européen des brevets**

(11) **EP 2 127 665 A1**

(12) **EUROPEAN PATENT APPLICATION**
published in accordance with Art. 153(4) EPC

(43) Date of publication:
**02.12.2009 Bulletin 2009/49**

(51) Int Cl.:
*A61K 38/02* (2006.01)   *A61P 5/50* (2006.01)

(21) Application number: **07808742.6**

(22) Date of filing: **16.07.2007**

(86) International application number:
**PCT/RU2007/000387**

(87) International publication number:
**WO 2008/091177 (31.07.2008 Gazette 2008/31)**

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **18.01.2007  RU 2007101695**

(71) Applicant: **Otkrytoe Aktsionernoe Obschestvo "Otechestvennye Lekarstva"**
**Moscow 129090 (RU)**

(72) Inventors:
• **NESTERUK, Vladimir Viktorovich**
 **Moscow, 125171 (RU)**

• **SYROV, Kirill Konstantinovich**
 **Moscow, 129110 (RU)**
• **TITOV, Mikhail Ivanovich**
 **St.Petersburg, 198504 (RU)**
• **VINOGRADOV, Valentin Antonovich**
 **Moscow 123007 (RU)**
• **SERNOV, Lev Nikolaevich**
 **Moskovskaya oblast, 142440 (RU)**

(74) Representative: **Wiedemann, Peter et al**
**Hoffmann - Eitle**
**Patent- und Rechtsanwälte**
**Arabellastrasse 4**
**81925 München (DE)**

(54) **EXENATIDE AND DALARGIN-BASED MEDICINAL PREPARATION FOR TREATING PANCREATIC DIABETES**

(57)    This invention relates to the field of medicine, in particular, to endocrinology. This invention offers a method of potentiation of a therapeutic effect of exenatide by administration of an effective quantity of exenatide in combination with an effective quantity of dalargin. Advantageous therapeutic effects of the invention include lowering of pathologically elevated levels of blood glucose and cholesterol in patients suffering from diabetes mellitus. This invention offers a method of treatment of diabetes mellitus by administration of an effective quantity of exenatide in combination with an effective quantity of dalargin. This invention offers the use of the combination of exenatide and dalargin for the preparation of the medication for the treatment of diabetes mellitus. This invention offers a medicinal preparation for the treatment of diabetes mellitus containing an effective quantity of exenatide, an effective quantity of dalargin and a pharmaceutically acceptable vehicle or diluent, as well as a method of preparation of this preparation. This invention offers a medicinal preparation for the treatment of diabetes mellitus in the form of a kit containing the medicinal preparation exenatide and the medicinal preparation dalargin, as well as a method of use of this medicinal preparation.

EP 2 127 665 A1

**Description**

This invention relates to the field of medicine, in particular, to endocrinology, namely, to medications for the treatment of diabetes mellitus.

Diabetes mellitus presents a group of metabolic diseases characterized by pathologically elevated levels of blood glucose which occur owing to defects in insulin secretion, insulin action or both defects. A the end of 1999, the number of patients suffering from diabetes mellitus in the world amounted to 120-140 mln., and this number may double by 2025. Diabetus mellitus, WHO information, Fact Sheet № 138, Reviewed November (1999). Diagnostic criterion of diabetes mellitus is an elevated concentration of glucose in plasma of venous and capillary blood after an overnight fast > 7.8 mmol/l or in whole venous or capillary blood > 6.7 mmol/l; in 2 h after load of 75 g of glucose the level of glucose in plasma of venous blood > 11.1 mmol/l (200 mg/100 ml) and in plasma of capillary blood > 12.2 mmol/l (220mg/100 ml); in whole venous blood > 10.0 (180mg/100 ml) and in whole capillary blood > 11.1 mmol/l (200 mg/100ml). The main goal of treatment of diabetes mellitus consists in the maintenance of the normal level of blood glucose. For this purpose, various medications are used, for instance, sulfonylurea, metformin and insulin. Exenatide is one of medications, which efficiency in treatment of diabetes mellitus, both insulin-dependent and insulin-independent, has been proved in clinical trials on humans.

Exenatide is a polypeptide also known under the name of exendin-4, the isolation technique of which from the venom of the lizard *Heloderma suspectum* and the amino acid sequence HGEGTFTSDL SKQMEEEAVR LFIEWLKNGG PSSGAPPPS, were first published in April 1992. Eng. J. et al., Isolation and Characterization of Exendin-4. an Exendin-3 Analogue, from Heloderma suspectum Venom. J. Biol. Chem.. Vol, 267, Issue 11, 7402-7405. Apr, 1992.

Patent US No.5424286, the priority date May 24 1993, the claim 6, discloses the use of exenatide (exendin-4) to stimulate insulin secretion with an greater efficiency than the incretin hormone GLP-1. Description of invention states that exenatide is useful for the treatment of diabetes mellitus type 2 (insulin-independent) and diabetes mellitus type 1 (insulin-dependent).

Later on other therapeutic effects of exenatide were discovered. Patent Application US2005043238 describes the use of exenatide to reduce weight. International publication WO9805351 and Patent US 6858576 disclose the use of exenatide to slow-down gastric motility and gastric emptying in therapeutic and diagnostic purposes. Publications US2005037958, US6703359, WO9940788 disclose an inotropic and diuretic action of exenatide. International publications WO3061362, WO2004052390 and Patent Application US2004266678 describe the use of exenatide for the treatment of polycystic ovary disease. International publication WO2004056317 and Patent Application US2004209803 disclose the use of exenatide for the treatment and prophylaxis of nephropathy. Publication WO2004056313 discloses the use of exenatide for the prophylaxis and treatment of cardiac arrhythmia. Publication WO2004035623 describes stabilized combinations of exenatide. Publication WO2004016747 discloses the use of exenatide to differentiate bone marrow cells. Publication WO0073331 and Patent Application US2004023871 disclose the use of exendin and its agonists for the treatment of diabetes mellitus in pregnancy. Patent Application US2003087821 discloses new usages of exendin, exendin agonists. Publication WO0151078 and Patent Application US2003036504 discloses the use of exenatide to control the level of triglycerides and to treat dyslipidemia. Publication WO0054148 and patent RU2247575 disclose the use of exenatide to suppress glucagon. Publication WO0009666 discloses the use of exenatide to differentiate non-insulin producing cells into insulin producing. Patent EP1419783 discloses new usages of exendin. Publication WO9907404 discloses the use of exenatide, exenatide agonists and antagonists to impact the central nervous system. Publication WO9830231 and Patent Application US2002137666 disclose the use of exenatide and its agonists to decrease meal absorption.

Exenatide is primarily used for the treatment of diabetes mellitus both insulin-independent and insulin-dependent. However, its use is accompanied by a series of essential drawbacks, such as its frequent application in the form of injections (multiple injections per day every day), a number of side effects caused by the isolation of exenatide out of the monster lizard's venom, and a high cost of exenatide. Side effects of exenatide treatment include, for instance, individual intolerance and nausea. Besides, the therapeutic efficacy of exenatide is limited by its high toxicity, and the achievement of an efficient reduction (normalization) of blood glucose, the first and foremost goal of the treatment of diabetes mellitus, is limited by the impossibility to use high toxic doses of exenatide. Therefore, there remains a challenge to create new exenatide preparations with higher efficacy, at lower cost and without side effects of exenatide. There are two primary approaches to this problem.

The first approach is associated with production and use of new peptides-analogs of exenatide and modified exenatides. Publication W03099314, Patent Application AU20033239910, JP2004506838 describe new exenatide (exendin) agonists. Publication WO0066629 and Patent CN1372570 describe modified exendin and exendin agonists. Publication WO0041546 and Patent CN1284755 describe new combinations of exendin agonists. Patent Application US2004242853 describes new peptide analogs of Glp-1 and Exenatide. Publication W002090388 and Patent Application US2004142866 describe new derivatives of insulinotropic peptide exendin-4 (exenatide). Patent Application US2004092443 describes exendin and exendin agonists with prolonged action. Patent Application US2004106547 describes new peptide-agonists with GLP-1 and exenatide activity. Patent Publication WO03011892 describes new analogs of GLP-1 and exenatide peptide. Patent Application US2003087820 describes new medicinal combinations of exendin agonists. Patent CN1381271 describes a new analog of exenatide. Publication WO0216430 and Patent Application US2002115605 describe a new analog of exenatide with a cerebral action. Patent EP1196444 describes new peptides, analogs of exenatide used to reduce the blood glucose level. Patent EA3922 describes new prolonged-action insulinotropic peptides, exenatide analogs. Patent EP 1076066 describes new peptides used to reduce the blood glucose level, exenatide analogs. Patent US6767887 describes new analogs of exenatide for the treatment of diabetes mellitus. Patent EP1056775 describes new derivatives of exenatide. Patent Application KR2000016389 describes new analogs of exenatide. Publications WO9925728, W09925727 describe new combinations of exenatide agonists. Patent DE19637230 describes new varieties of exenatide for the treatment of diabetes.

The second approach is associated with the discovery and use of new pharmaceutical combinations of exenatide with another active component in a pharmaceutical composition targeted to raise the efficiency of treatment, decrease the efficient dose of exenatide and, therefore, to reduce side effects connected with a necessity to use higher doses of exenatide, and to lower the cost of treatment. Publication WO200500022 discloses new means and compositions for the improved delivery of peptides and proteins, including exenatide, via mucosa. Patent Application US2004266692 discloses new pharmaceutical compositions of exendin. Publication WO2004050115 and Patent Application US2004180824 disclose the use of exenatide for the treatment in combination with tiazolidindions. Patent RU2242244 discloses the use of exenatide compositions for the treatment of diabetes mellitus, the slow-down of stomach emptying or the reduction of food consumption. Patent CA2389462 discloses new compositions of exenatide for the prophylaxis of diabetes.

We have established that joint administration of exenatide and dalargin oligopeptide with the amino acid sequence - tirozil-2-alanil-glycil-phenylalanyl-leucil-arginine - to mammals solves the task of creation of a medication possessing an increased therapeutic efficacy with a simultaneous decrease of side effects, which is the goal of this invention.

Dalargin in said combination potentiates therapeutic effects of exenatide, i.e., a desired therapeutic effect is achieved with smaller quantities of exenatide, and, therefore, said combination decreases side effects associated with the use of large doses of exenatide, and cuts the cost of the medication as well. Moreover, said combination enables to reach greater absolute effect as compared with that reached when using exenatide alone, as the achievement of this greater effect is limited by exenatide toxicity and is possible only with the use of greater than pharmacologically acceptable toxic doses of exenatide. Thus, with the use of said combination better results are reached in the maintenance of the normal level of blood glucose as compared with those which can be achieved with the use of exenatide alone.
As of today, the use of dalargin in combination with Exenatide is unknown.

From the state of art, the use of dalargin as a means to reduce pathologically elevated levels of blood glucose, which is the primary goal of treatment of diabetes mellitus, remains undisclosed. The main known properties of dalargin are related to its efficacy as a protector protecting the bodily cells from damage under unfavorable conditions, including in various diseases. It is known that dalargin is used for the treatment of diabetes complications. Patent RU2270025 describes the use of dalargin for the treatment of diabetic retinopathy. Patent RU2144831 discloses the use of dalargin for the treatment of a patient with a diabetic foot syndrome. It is clear that the treatment of diabetes mellitus complications is not identical to the treatment of diabetes and sets a goal of treatment of organs and tissues damaged owing to the presence of diabetes, while the treatment of diabetes mellitus consists, first and foremost, in the achievement of best control over the blood glucose level. Thus, the use of dalargin in the treatment of diabetic retinopathy and diabetic foot known from the state of art is not the argument to state that dalargin can be used or was used already some time for the treatment of diabetes.

The usages of dalargin are known to be related, primarily, with a protective effect of dalargin, what does not negate novelty and inventive level of this invention.

Patent RU2266130 discloses the use of dalargin for the treatment of arrhythmic syndrome at progressing angina pectoris. Patent RU2261713 discloses the use of dalargin for the treatment of a severe form of acute optical neutritis in a patient with disseminated sclerosis. Patent RU2261722 discloses the use of dalargin for the treatment of a latent form of genital herpes in a female with a fetal death syndrome. Patent RU2254890 discloses the use of dalargin to stimulate gastric motility in patients with vertebral column traumas. Patent RU2261721 discloses the use of dalargin for intraoperative neuroprotection in patients with cerebral arterial aneurisms. Patent RU2258529 discloses the use of dalargin for early treatment of degree II and III hemorrhagic shock. Patent Application RU2004100992 discloses the use of dalargin for endoscopic hemostasis in case of gastroduodenal ulcers complicated by hemorrhage. Patent UA7497U discloses the use of dalargin for the treatment of primarily diagnosed duodenal ulcer in patients of excitable personality type. Patent UA 6829U discloses the use of dalargin for the treatment of acute experimental pancreatitis. Patent UA6826U discloses the use of dalargin for the treatment of acute experimental pancreatitis. Patent UA6823U discloses the use of dalargin for the treatment of acute experimental pancreatitis. Patent UA6576U discloses the use of dalargin for stabilization of remission in patients with opium addiction. Patent UA67632 discloses the use of dalargin for correction of stress on an experimental model of chronic pancreatitis. Patent UA67631 discloses the use of dalargin for correction of stress on an experimental model of acute pancreatitis induced by L- arginine. Patent UA67630 discloses the use of dalargin for correction of stress on an experimental model of acute adnexitis. Patent UA67629 discloses the use of dalargin for correction of stress on an experimental model of peritonitis. Patent UA67626 discloses the use of dalargin for correction of experimental chronic stress. Patent RU2241488 discloses the use of dalargin for preparation of a solution for injections. Patent UA67601 discloses the use of dalargin to stimulate adhesion in bone fractures. Patent RU2228762 discloses the use of dalargin for early therapy of gastrointestinal hemorrhages. Patent UA61455 discloses the use of dalargin for the prophylaxis of early formation of postoperative intra-abdominal adhesions. Patent UA61449 discloses the use of dalargin for the prophylaxis of formation of postoperative intra-abdominal adhesions. Patent RU2228148 discloses the use of dalargin for post-operative anesthesia of patients with necrotizing pancreatitis after omentobursostoma with multiple programmed sanation relaparotomy performances. Patent RU2218896 discloses the use of dalargin for the treatment of post-inflammation bullous keratopathy. Patent RU2217139 discloses the use of dalargin to stimulate gastric motility. Patent RU2217186 discloses the use of dalargin for the treatment of benign lesions to uterine cervix. Patent RU2230549 discloses the use of dalargin for the treatment of allergic dermatoses. Publication MD1963F discloses the use of dalargin for the treatment of chronic recurrent aphthous stomatitis. Patent RU2200026 discloses the use of dalargin as an anti-anginal means. Patent RU2180598 discloses the use of dalargin for the treatment of toxic hepatitis in patients with chronic drug addiction. Patent RU2185176 discloses the use of dalargin for the treatment of dystrophic diseases of the cornea and corneal transplant disease. Publication MD1610F discloses the use of dalargin for the treatment of recurrent chronic aphthous stomatitis. Patent RU2164153 discloses the use of dalargin for the treatment of dumping syndrome. Patent RU2196603 discloses the use of dalargin for the treatment of bum disease. Patent RU2188675 discloses the use of dalargin for the treatment of patients with amenorrhea of central genesis. Patent RU2185849 discloses the use of dalargin for the treatment of opium abstinence symptoms. Patent RU2180591 discloses the use of dalargin for the treatment of sexual dysfunctions in males. Patent RU2167671 discloses the use of dalargin for the treatment of patients with mite encephalitis. Publication MD1413F discloses the use of dalargin for the treatment of oral mucosa and lichen planus. Patent RU2198641 discloses the use of dalargin for the treatment of eye diseases. Patent MD1296F discloses the use of dalargin for the treatment of lichen planus. Patent RU2181564 discloses the use of dalargin for metabolic correction in a combined intensive therapy of hematogenic shock. Patent RU2146530 discloses the use of dalargin for anesthesia in surgical operations on the brain. Publications CZ9602751, CZ9602627 disclose the use of glycolised analogs of dalargin. Patent RU2142736 discloses the use of dalargin for premedication. Patent RU2142814 discloses the use of dalargin as a means with cholelitolitic action. Patent RU2122415 discloses the use of dalargin for intensive therapy of patients with a traumatic shock. Patent RU2104717 discloses the use of dalargin for anesthesia in operations on patients with Leriche's syndrome. Publication CZ9301718 discloses analogs of dalargin with N-methylated peptide bond. Patent RU2113856 discloses the use of dalargin for the prophylaxis of relapses of anginal pains at myocardial infarction.

This invention offers a method for the potentiation of a therapeutic effect of exenatide, characterized in that the mammal in need thereof receives an effective quantity of exenatide in combination with an effective quantity of dalargin.

The term "the potentiation of a therapeutic effect" means that a therapeutic effect reached by the administration of exenatide to a mammal in combination with an effective quantity of dalargin, is greater than a therapeutic effect reached by the administration of an equivalent quantity of exenatide, but without dalargin, and at all other things being equal.

A positive effect of the combination comprising exenatide with dalargin is associated with an improved functioning of the pancreas owing to the protective action of dalargin, what is experimentally proved by us. Due to better functioning of the pancreas, an effective control of blood glucose is reached at smaller doses of exenatide, when exenatide is used in combination with dalargin.

The nearest analog of this invention is the combination described in the international publication WO2004050115, where an improved control of the blood glucose level in patients suffering from diabetes mellitus was achieved with the use of the combination comprising two active agents, exenatide and an agent out of the class of tiazolidindions. Both active ingredients are known as the agents that decrease pathologically elevated blood glucose levels in patients suffering from diabetes, but the combination of these agents enables to achieve a better control of glucose as compared with each ingredient of the combination individually. However, a protector effect of this combination in respect of pancreatic cells is unknown.

Owing to potentiation of the therapeutic effect of exenatide, the therapeutic effect of exenatide in combination with dalargin may be achieved with a smaller quantity of exenatide as compared with usual therapeutic doses. Therefore, the use of this invention presents a possibility to minimize potential side effects associated with the use of large therapeutic doses of exenatide, and at the same time to achieve the therapeutic effect at a lower cost of treatment. Examples of therapeutic effects of exenatide are well known from the state of art, and include also a decrease of pathologically elevated blood glucose levels in patients suffering from diabetes mellitus, weight loss, slow-down of gastric motility and gastric emptying in therapeutic and diagnostic purposes, inotropic and diuretic action of exenatide, improved differentiation of bone marrow cells, modulation of the level of triglycerides and treatment of dyslipidemia, suppression of glucagons, differentiation of non-insulin-producing cells into insulin-producing, the effect upon the central nervous system and a decrease in meal absorption.

Advantageous therapeutic effects of exenatide, according to this invention, include a decrease in pathologically elevated levels of glucose and cholesterol in blood of patients with diabetes.

Further, this invention offers a method of therapy of diabetes mellitus, characterized in that a mammal in need thereof receives an effective quantity of exenatide in combination with an effective quantity of dalargin. Preferentially, the mammal is a human being.

The term "an effective quantity" means a quantity of exenatide or dalargin sufficient to achieve a desired therapeutic effect but insufficient to cause side effects in the mammal, preferentially a human being in need of such treatment. "An effective quantity" appears to vary dependent on such factors as the state of the mammal, duration of treatment, a specific dosage form used. Preferentially, an effective quantity of exenatide varies from 0.03 to 7.5 mcg/kg of mammalian body mass, more preferentially from 0.07 to 0.36 mcg/kg of mammalian body mass. Preferentially, an effective quantity of dalargin in a single dosage form of the medication varies from 0.14 to 14.3 mcg/kg of mammalian body mass, more preferentially from 0.14 to 0.71 mcg/kg of mammalian body mass.

According to this invention, an effective quantity of exenatide in combination with an effective quantity of dalargin may be administered to the mammal using various routes, for instance, subcutaneous injections, intramuscular injections, via lungs, intranasally and sublingually. Preferentially, an effective quantity of exenatide in combination with an effective quantity of dalargina is administered parenterally, for instance, subcutaneously.

Further, this invention suggests that a combination comprising exenatide and dalargin be used to produce a medication for the treatment of diabetes mellitus in the mammal in need thereof.

Further, this invention offers a medication based on the combination of exenatide и dalargina for the treatment of diabetes mellitus in the mammal in need thereof.

Further, this invention offers a medicinal preparation in a dosage form for the treatment of diabetes mellitus, which contains an effective quantity of exenatide and a pharmaceutically acceptable vehicle or diluent distinguished in that it additionally contains an effective quantity of dalargin.

According to this invention, the medicinal preparation is produced in a dosage form. Preferentially, an effective quantity of exenatide in a single dose varies from 2 to 100 mcg, more preferentially, from 5 to 25 mcg. Preferentially, an effective quantity of dalargin in a single dose varies from 10 to 1000 mcg, more preferentially, from 10 to 50 mcg. Preferentially, the dosage form contains 60 single doses.

The term "a single dose" means that a quantity of a medication is administered single-shot to the mammal in need thereof.

According to this invention, the medication contains a pharmaceutically acceptable vehicle or diluent. The term "a pharmaceutically acceptable vehicle or diluent" means one or several compatible liquid or solid excipients or diluents, which are suitable for administration to a mammal preferentially a human being. The term "compatible" means that the vehicle or diluent may be mixed with exenatide, dalargin and auxiliary ingredients of the medication without inducing any interaction that can decrease the therapeutic efficiency of the medication. Instances of agents that can serve as pharmaceutically acceptable vehicles or diluents include water for injections, isotonic solution of salt, solutions of phosphate buffers, lactose, propylene glycol, glycerin, sorbit, mannit, coloring agents, emulsifiers, preservatives, stabilizers and antioxidants. Water for injections serves as a preferential vehicle or diluent in this invention.

According to this invention, the medicinal preparation may be prepared in various dosage forms, for instance, liquid, solid or gaseous suitable, for example, for parenteral, intranasal, oral, oromucosal or pulmonary administration. Examples of liquid dosage forms include solutions, suspensions, emulsions and drops. Examples of solid dosage forms include tablets, powders and capsules. Examples of gaseous dosage forms include aerosols. The preferential dosage form of this invention is water for injections.

This invention offers a method for preparation of the medicinal preparation for the treatment of diabetes mellitus, characterized by that exenatide and dalargin are mixed with a pharmaceutically acceptable vehicle or diluent, and this mixture is placed into the fit-for-use form. Preferentially, exenatide and dalargin together with auxiliary ingredients, for instance, buffers, stabilizers, osmolarity moderators and preservatives are dissolved in water for injections and poured into ampoules under sterile conditions with each ampoule containing a therapeutically effective quantity of exenatide and dalargin for a single administration to the mammal in need thereof.

According to this invention, the medicinal preparation may be administered by various routes. Some examples of such routes include intramuscular, subcutaneous or intravenous injections, as well intranasal, transdermal, pulmonary, sublingual, rectal or peroral administration. Subcutaneous injection presents a preferential route for administration of the medication to the mammal in need thereof.

Further, this invention offers the medicinal preparation presenting a kit for the treatment of diabetes mellitus and is characterized by containing: (a) the first medicinal preparation in a dosage form containing an effective quantity of lyophilized powder of exenatide and a pharmaceutically acceptable solid vehicle or diluent; and (6) the second medicinal preparation in a dosage form containing an effective quantity of dalargin solution for injections.

Preferentially, an effective quantity of exenatide in a single dose of the first medicinal preparation varies from 2 to 100 mcg, more preferentially, from 5 to 25 mcg. Preferentially, an effective quantity of dalargin in a single dose of the second medicinal preparation varies from 10 to 1000 mcg, more preferentially, from 10 to 50 mcg. Preferentially, the dosage forms of the first and second medicinal preparation contain 60 single doses.

This invention offers a method of use of the medicinal preparation presenting a kit for the treatment of diabetes mellitus and characterized by containing: (a) the first medicinal preparation in a dosage form containing an effective quantity of lyophilized powder of exenatide and a pharmaceutically acceptable solid vehicle or diluent; and (6) the second medicinal preparation in a dosage form containing an effective quantity of dalargin solution for injections. Preferentially, the second medicinal preparation contains a pharmaceutically acceptable antiseptic that is meta-cresol. Said method of use is characterized by that a single dose of the first medicinal preparation is mixed with a single dose of the second medicinal preparation to form a homogenous solution and the obtained solution is administered subcutaneously to the mammal in need thereof.

This invention offers one more method of use of the medicinal preparation presenting a kit for the treatment of diabetes mellitus. Said method of use is characterized by that 60 single doses of the first medicinal preparation are mixed with 60 single doses of the second medicinal preparation to form a homogenous solution, and 1/60th part of the obtained solution is administered twice daily for 30 days subcutaneously to the mammal in need thereof.

Preferentially, the mammal is a human being.

The following examples demonstrate the invention. Examples are presented only for illustration and in no way limit the boundaries of the invention claims.

Example 1

This example demonstrates a method for the potentiation of therapeutic effects of exenatide and a method of treatment of diabetes mellitus.

Diabetes was induced in white mice by a single intraperitoneal administration of alloxan in a dose of 135 mg/kg. Exenatide in a dose of 7.5 mcg/kg, dalargin in a dose of 8.8 mcg/kg or a combination of exenatide in a dose of 7.5 mcg/kg plus dalargin in a dose of 8.8 mcg/kg were administered subcutaneously to mice twice daily for 30 days. Control animals received injections of saline solution. By day 31 of the experiment, the levels of blood glucose and cholesterol were measured after an overnight fast. Data are presented as a mean $\pm$ standard deviation (n=5) of the measured levels of glucose or cholesterol. The therapeutic effect $\Delta$ was estimated as a difference between an observed mean value of the observed parameter in experimental animals and a mean value of the same parameter in control animals. To determine the therapeutic effect of lowering the level of glucose, the following formula was used:

$$\Delta\, \text{Glucose} = \text{Glucose}_{\text{medication}} - \text{Glucose}_{\text{control}}.$$

To determine the therapeutic effect of lowering the level of cholesterol, the following formula was used:

$$\Delta\, \text{Cholesterol} = \text{Cholesterol}_{\text{medication}} - \text{Cholesterol}_{\text{control}}.$$

Statistical differences of the obtained results were assessed with the use of Student's t-test. Data are given in tables 1 and 2.

Table 1

| Blood glucose level in mice with alloxan diabetes | | |
|---|---|---|
| Medication | Glucose, mmol/l | $\Delta$ Glucose, mmol/l |
| Control | 11.5 $\pm$ 0.9 | 0 |
| Exenatide | 7.5 $\pm$ 0.4* | -4.0 |
| Dalargin | 10.6 $\pm$ 1.3 | -0.9 |

(continued)

| Blood glucose level in mice with alloxan diabetes | | |
|---|---|---|
| Medication | Glucose, mmol/l | Δ Glucose, mmol/l |
| Exenatide + Dalargin | 4.2 ± 0.9* | -7.3 |
| *Statistically significant difference from control ($p < 0.05$). | | |

Mice with alloxan diabetes had significantly elevated blood glucose levels after an overnight fast as compared with intact mice (11.5 ± 0.9 vs. 5.3 ± 0.3 mmol/l, $p < 0.05$). Exenatide and the combination of "exenatide + dalargin" significantly lowered blood glucose levels after an overnight fast in mice with alloxan diabetes. However, the effect of the combination was essentially greater (a reduction by 7.3 mmol/l), than the effect of exenatide (a reduction by 4.0 mmol/l), taken in the quantity equivalent to that used in the combination "exenatide + dalargin", but without dalargin, at all other things being equal. Dalargin, taken separately in the quantity equivalent to that used in the combination "exenatide + dalargin" was inefficient in lowering blood glucose levels in mice with alloxan diabetes. Thus, dalargin potentiates the therapeutic effect of exenatide, associated with lowering pathologically elevated levels of blood glucose. Correspondingly, the treatment of diabetes mellitus by joint administration of efficient quantities of exenatide and dalargin has an advantage before monotherapy of diabetes mellitus by administration of exenatide.

Table 2

| Blood cholesterol level in mice with alloxan diabetes | | |
|---|---|---|
| Medication | Cholesterol, mmol/l | Δ Cholesterol, mmol/l |
| Control | 2.61 ± 0.25 | 0 |
| Exenatide | 2.15 ± 0.22* | -0.46 |
| Dalargin | 2.34 ± 0.16 | -0.27 |
| Exenatide + Dalargin | 1.46 ± 0.12* | -1.15 |
| * Statistically significant difference from control ($p < 0.05$). | | |

Mice with alloxan diabetes had significantly elevated levels of blood cholesterol after an overnight fast as compared with intact mice (2.61 ± 0.25 vs. 1.02 ± 0.37 mmol/l, $p < 0.05$). Exenatide and combination "exenatide + dalargin" significantly lowered the level of cholesterol after an overnight fast in mice with alloxan diabetes. However, the effect of the combination was essentially greater (a reduction by 1.15 mmol/l), than the effect of exenatide (a reduction by 0.46 mmol/l) taken in the quantity equivalent to that use in the combination "exenatide + dalargin", but without dalargin, at all other things being equal. Dalargin, taken separately in the quantity equivalent to that use in the combination "exenatide + dalargin", was inefficient in lowering blood cholesterol in mice with alloxan diabetes. Thus, dalargin potentiates the therapeutic effect of exenatide associated with lowering pathologically elevated levels of blood cholesterol.

Lesions and death of pancreatic beta-cells in experimental mice were evaluated morphometrically. The share of normal intact cells in mice in the control group, mice treated with exenatide, and mice treated with the combination of exenatide with dalargin was approximately 8, 40, and 60%, respectively. Thus, the combination of exenatide with dalargin essentially improves the survival and functioning of pancreatic cells as compared with exenatide administered alone in the same dose that was used in combination, at all other things being equal.

Example 2

This example demonstrates the method of treatment of diabetes mellitus.

Diabetes was induced in mice in the same way as in experiment 1. Exenatide in various doses, dalargin in various doses or a combination of exenatide with dalargin were administered subcutaneously to mice twice daily for 30 days. Control animals received saline injections. On day 31 of the experiment blood glucose levels after an overnight fast were measured. Data are given in Table 3 as an average reduction of glucose level after an overnight fast (Δ Glucose) on day 31 of the experiment in mice (n=5) with alloxan diabetes treated with a combination of exenatide and dalargin as compared with animals treated with exenatide alone taken in an equivalent dose and at all other things being equal. To determine the average reduction of the glucose level the following formula was used:

$$\Delta \text{ Glucose} = \text{Glucose}_{\text{Exenatide}} - \text{Glucose}_{\text{Exenatide + dalargin}}.$$

Table 3

| Average lowering of blood glucose level ($\Delta$ Glucose) in mice with alloxan diabetes treated with the combination of efficient quantities of exenatide and dalargin | | |
|---|---|---|
| Components of the combination "exenatide + dalargin" | | $\Delta$ Glucose, mmol/l |
| Exenatide, mcg/kg | Dalargin, mcg/kg | |
| 0.03 | 14.3 | -5.3 |
| 1.4 | 0.14 | -2.8 |
| 7.5 | 8.8 | -3.3 |

The table demonstrates that blood glucose level after an overnight fast in mice with alloxan diabetes treated for 30 days with a combination of exenatide in a dose of 0.03 mcg/kg and dalargin in a dose of 14,3 mcg/kg is by 5.3 mmol/l lower ($\Delta$ Glucose = - 5.3 mmol/l) as compared with blood indices of mice treated with exenatide alone in a dose of 0.03 mcg/kg. The level of blood glucose after an overnight fast in mice with alloxan diabetes treated for 30 days using a combination of exenatide in a dose of 1.4 mcg/kg and dalargin in a dose of 0.14 mcg/kg is by 2.8 mmol/l lower as compared with blood indices in mice treated with exenatide alone in a dose of 1.4 mcg/kg. The level of blood glucose after an overnight fast in mice with alloxan diabetes treated for 30 days using a combination of exenatide in a dose of 7.5 mcg/kg and dalargin in a dose of 8.8 mcg/kg is by 3.3 mmol/l lower as compared with blood indices in mice treated with exenatide alone in a dose of 7.5 mcg/kg. Thus, the combination of exenatide with dalargin is essentially more effective for the treatment of diabetes mellitus as compared with an equal dose of exenatide, taken in said combination, if exenatide is used separately without dalargin.

Example 3

This example demonstrates the use of the combination comprising exenatide and dalargin to prepare the medication and medicinal preparation for the treatment of diabetes mellitus.

Load 750 ml water for injections, mannit 40.15 g, meta-cresol 3.3 g into a flask with an agitator, stir the mass, add 10 ml of buffer with pH 4.7-7.0 and a combination containing exenatide 2-100 mg and dalargin 10-1000 mg. Stir the solution, add water for injections to bring the volume up to 1 1. Pass the solution using sterilizing filtration consequentially through filters with a 0.45 $\mu$m and 0.22 $\mu$m poresize. The obtained solution for injections is poured under sterile conditions into ampoules by 1 ml to obtain 950 ampoules. The solution complies with requirements of State Pharmacopoeia XI and normative documentation for a medicinal preparation for injections. The composition of the preparation is given in Table 4.

Table 4

| Ingredient | Quantity per ampoule |
|---|---|
| Exenatide | 2-100 mcg |
| Dalargin | 10-1000 mcg |
| Mannit | 40 МГ |
| Preservative | 33 МГ |
| Buffer mixture | 0.15 МГ |
| Water for injections | 1 MJI |

Ampoules with the preparation for injections are packed into a suitable package. Each package contains 10 ampoules and instruction for use of the medicinal preparation for the treatment of diabetes mellitus.

Method of use: the contents of an ampoule (a single dose of the preparation for the treatment of diabetes mellitus) are administered subcutaneously to a human in need thereof. Administration may be repeated during 24 h. The course of treatment may be one day or longer.

Example 4

This example demonstrates a medicinal preparation for the treatment of diabetes mellitus.

Solution for injections comprising exenatide and dalargin is prepared as stated in Example 3. Solution for injections containing 60 single doses is placed into a flask suitable for multiple retrieval of doses.

Example 5

This example demonstrates the medicinal preparation in a kit for the treatment of diabetes mellitus and a method of its use.

Preparation of the first medicinal preparation. Pour 1000 ml of water for injections, 40 Γ of mannit, 2-100 mg of exenatide, stir the mass to form a homogenous solution. Pass the solution using sterilizing filtration consequentially through filters with a 0.45 μm and 0.22 μm poresize. The obtained solution for injections is poured under sterile conditions into flasks and is lyophilized to obtain 950 flasks. One flask contains 2-100 mcg of lyophilized powder of exenatide and 20 mg of mannit as a pharmaceutically acceptable solid vehicle or diluent.

Preparation of the second medicinal preparation. Pour 1000 ml of water for injections, 10-1000 mg of dalargin, 150 mg of meta-cresol, 830 mg of succinic acid, bring pH to 4.9-5.3 using sodium hydrate, stir the mass to form a homogenous solution. Pass the solution using sterilizing filtration consequentially through filters with a 0.45 μm and 0.22 μm poresize. The obtained solution for injections is poured under sterile conditions into flasks to obtain 950 flasks. One flask contains solution for injections containing 10-1000 mcg of dalargin.

First and second medicinal preparations are packaged into a suitable pack. Each pack contains 10 flasks of each preparation and instruction for use of the medicinal preparation for the treatment of diabetes mellitus.

Method of use: the contents of the first flask are dissolved in the contents of the second flask and the obtained homogenous solution is administered single-shot subcutaneously to a human being suffering from diabetes mellitus.

Example 6

This example demonstrates the medicinal preparation in a kit for the treatment of diabetes mellitus and a method of its use.

Preparation of the first medicinal preparation. Pour 10 ml of water for injections, 50 mg of mannit, 0.12-6.0 mg of exenatide into a flask, stir the mass to form a homogenous solution. Pass the solution using sterilizing filtration consequentially through filters with a 0.45 μm and 0.22 μm poresize. The obtained solution for injections is poured under sterile conditions into flasks and is lyophilized. One flask contains 0.12-6.0 Mmg of lyophilized powder of exenatide and 50 mg of mannit as a pharmaceutically acceptable solid vehicle or diluent.

Preparation of the second medicinal preparation. Pour 3 ml of water for injections, 0.6-60 mg of dalargin, 9 mg of meta-cresol, 50 mg of succinic acid, bring pH to 4.9-5.3 using sodium hydrate, stir the mass to form a homogenous solution. Pass the solution using sterilizing filtration consequentially through filters with a 0.45 μm and 0.22 μm poresize. The obtained solution for injections is poured under sterile conditions into a flask. One flask contains dalargin solution for injections containing 0.6-60 mg of dalargin.

First and second medicinal preparations are packaged into a suitable pack. Each pack contains one flask of each preparation and instruction for use of the medicinal preparation for the treatment of diabetes mellitus.

Method of use: the contents of the first flask are dissolved in the contents of the second flask and 1/60th of the obtained homogenous solution is administered twice daily for 30 days subcutaneously to a human being suffering from diabetes mellitus.

**Claims**

1. The method for potentiating the therapeutic effect of exenatide, **characterized in that** a mammal needing thereof receives an effective quantity of Exenatide in combination with an effective quantity of dalargin.

2. The method of claim 1, distinguished in that the therapeutic effect presents lowering of pathologically elevated blood glucose levels.

3. The method of claim 1, distinguished in that the therapeutic effect presents lowering of pathologically elevated blood cholesterol levels.

4. The method of treatment of diabetes mellitus, **characterized in that** a mammal needing thereof receives an effective quantity of exenatide in combination with an effective quantity of dalargin.

5. The method of claim 4, distinguished in that an effective quantity of exenatide varies from 0.03 to 7.5 mcg/kg of mammalian body mass.

6. The method of claim 4, distinguished in that an effective quantity of dalargin varies from 0.14 to 14.3 mcg/kg of mammalian body mass.

7. The method of claim 4, distinguished in that an effective quantity of exenatide in combination with an effective quantity of dalargin is administered parenterally.

8. The method of claim 4, distinguished in that the mammal is a human being.

9. The use of the combination comprising exenatide and dalargin for the production of the medication for the treatment of diabetes mellitus in the mammal in need thereof.

10. The medication based on combination of exenatide and dalargin for the treatment of diabetes mellitus in the mammal in need thereof.

11. The medicinal preparation in the dosage form for the treatment of diabetes mellitus comprising an effective quantity of exenatide and a pharmaceutically acceptable vehicle or diluent distinguished in that it additionally contains an effective quantity of dalargin.

12. The medicinal preparation according to claim 11, distinguished in that an effective quantity of exenatide in a single dose varies from 2 to 100 mcg.

13. The medicinal preparation according to claim 11, distinguished in that an effective quantity of dalargin in a single dose varies from 10 to 1000 mcg.

14. The medicinal preparation according to claim 11, distinguished in that the dosage form contains 60 single doses.

15. The medicinal preparation according to claim 11, distinguished in that water for injections is a pharmaceutically acceptable vehicle or diluent.

16. The method of preparation of the medicinal preparation or the treatment of diabetes mellitus, **characterized in that** exenatide and dalargin are mixed with a pharmaceutically acceptable vehicle or diluent and this mixture is placed into a suitable-for-use form.

17. The medicinal preparation, presenting a kit for the treatment of diabetes mellitus and **characterized by** containing:

   (a) the first medicinal preparation in a dosage form containing an effective quantity of lyophilized powder of exenatide and a pharmaceutically acceptable solid vehicle or diluent; and
   (6) the second medicinal preparation in a dosage form containing an effective quantity of dalargin solution for injections.

18. The medicinal preparation according to claim 17, distinguished in that an effective quantity of exenatide in a single dose of the first medicinal preparation varies from 2 to 100 mcg.

19. The medicinal preparation according to claim 17, distinguished in that an effective quantity of dalargin in a single dose of the second medicinal preparation varies from 10 to 1000 mcg.

20. The medicinal preparation according to claim 17, distinguished in that the dosage form of the first medicinal preparation contains 60 single doses.

21. The medicinal preparation according to claim 17, distinguished in that the dosage form of the second medicinal preparation contains 60 single doses.

22. The method of use of the medicinal preparation according to claim 17, **characterized in that** a single dose of the first medicinal preparation is mixed with a single dose of the second medicinal preparation to form a homogenous solution, and the obtained solution is administered in a single shot subcutaneously to the mammal in need thereof.

23. The method of use of the medicinal preparation according to claim 17, **characterized in that** 60 single doses of the first medicinal preparation are mixed with 60 single doses of the second medicinal preparation to form a homogenous solution, and 1/60th of the obtained solution is administered twice daily for 30 days to the mammal in need thereof.

24. The method of use according to claim 22 and 23, distinguished in that the mammal is a human being.

## INTERNATIONAL SEARCH REPORT

| | |
|---|---|
| | International application No. |
| | **PCT/RU 2007/000387** |

**A. CLASSIFICATION OF SUBJECT MATTER**

*A61K 38/02 (2006.01)*
*A61P 5/50 (2006.01)*

According to International Patent Classification (IPC) or to both national classification and IPC

**B. FIELDS SEARCHED**

Minimum documentation searched (classification system followed by classification symbols)

**A61K 38/01, A61P 5/50**

Documentation searched other than minimum documentation to the extent that such documents are included in the fields searched

Electronic data base consulted during the international search (name of data base and, where practicable, search terms used)

http://www.fips.ru, http://www.espacenet.com., http://www.pubmed.com., http://www.uspto.gov.,

http://www.eapatis.com., http://www.wipo.int, http://yandex.com., http://rambler.ru

**C. DOCUMENTS CONSIDERED TO BE RELEVANT**

| Category* | Citation of document, with indication, where appropriate, of the relevant passages | Relevant to claim No. |
|---|---|---|
| A | US 2006189535 A (KAUDSEN LOTTE B) 24.08.2006,the abstract | 1-24 |
| A | RU 2144831 C1 (DALNEVOSTOCHNY GOSUDARSTVENNY MEDITSINSKY UNIVERSITET) 27.01.2000, the abstract | 1-24 |
| A | UA 60999 A (ODESSKY GOSUDARSTVENNY MEDITSINSKY UNIVERSITET) 15.10.2003, the abstract | 1-24 |
| A | AMETOV A.S. "Perspektivy razvitiya diabetologii". Terapevtichesky arkhiv 2005, Vol. 77, No10, pages 5-9, found on the Internet on 12.11.2007, at http://hghltd.vzndex.net/yandbtm?url=http%3A%2F%www.voed.ra%2F persp razv | 1-24 |
| A | MIKHAIL N. "Exenatide: a novel apporoach for treatment of type 2 diabete", South Med J, 2006 Nov;99(I 1): 1271-9, thea abstract[found on 13.11.2007] found on the Internet MEDLINE PMID: 17195423 | 1-24 |

☐ Further documents are listed in the continuation of Box C.   ☐ See patent family annex.

| | |
|---|---|
| *   Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 13 November 2007 | 22 November 2007 |

| Name and mailing address of the ISA/ | Authorized officer |
|---|---|
| **RU** | |
| Facsimile No. | Telephone No. |

Form PCT/ISA/210 (second sheet) (July 1998)

EP 2 127 665 A1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5424286 A
- US 2005043238 A
- WO 9805351 A
- US 6858576 B
- US 2005037958 A
- US 6703359 B
- WO 9940788 A
- WO 3061362 A
- WO 2004052390 A
- US 2004266678 A
- WO 2004056317 A
- US 2004209803 A
- WO 2004056313 A
- WO 2004035623 A
- WO 2004016747 A
- WO 0073331 A
- US 2004023871 A
- US 2003087821 A
- WO 0151078 A
- US 2003036504 A
- WO 0054148 A
- RU 2247575
- WO 0009666 A
- EP 1419783 A
- WO 9907404 A
- WO 9830231 A
- US 2002137666 A
- WO 3099314 A
- AU 20033239910
- JP 2004506838 B
- WO 0066629 A
- CN 1372570
- WO 0041546 A
- CN 1284755
- US 2004242853 A
- WO 02090388 A
- US 2004142866 A
- US 2004092443 A
- US 2004106547 A
- WO 03011892 A
- US 2003087820 A
- CN 1381271
- WO 0216430 A
- US 2002115605 A
- EP 1196444 A
- EA 3922
- EP 1076066 A
- US 6767887 B
- EP 1056775 A
- KR 2000016389
- WO 9925728 A
- WO 9925727 A
- DE 19637230
- WO 200500022 A
- US 2004266692 A
- WO 2004050115 A
- US 2004180824 A
- RU 2242244
- CA 2389462
- RU 2270025
- RU 2144831
- RU 2266130
- RU 2261713
- RU 2261722
- RU 2254890
- RU 2261721
- RU 2258529
- RU 2004100992
- UA 7497U
- UA 6829U
- UA 6826U
- UA 6823U
- UA 6576U
- UA 67632
- UA 67631
- UA 67630
- UA 67629
- UA 67626
- RU 2241488
- UA 67601
- RU 2228762
- UA 61455
- UA 61449
- RU 2228148
- RU 2218896
- RU 2217139
- RU 2217186
- RU 2230549
- RU 2200026
- RU 2180598
- RU 2185176
- MD 1610 F
- RU 2164153
- RU 2196603
- RU 2188675
- RU 2185849
- RU 2180591
- RU 2167671

- MD 1413 F
- RU 2198641
- MD 1296 F
- RU 2181564
- RU 2146530
- CZ 9602751
- CZ 9602627

- RU 2142736
- RU 2142814
- RU 2122415
- RU 2104717
- CZ 9301718
- RU 2113856

**Non-patent literature cited in the description**

- **Eng. J. et al.** Isolation and Characterization of Exendin-4. an Exendin-3 Analogue, from Heloderma suspectum Venom. *J. Biol. Chem.,* April 1992, vol. 267 (11), 7402-7405